(19) Europäisches Patentamt
European Patent Office
Office européen des brevets

(11) **EP 1 646 343 B1**

(12) **EUROPÄISCHE PATENTSCHRIFT**

(45) Veröffentlichungstag und Bekanntmachung des Hinweises auf die Patenterteilung:
**17.09.2014 Patentblatt 2014/38**

(21) Anmeldenummer: **04763195.7**

(22) Anmeldetag: **13.07.2004**

(51) Int Cl.:
*A61F 9/01* (2006.01)    *A61F 9/011* (2006.01)

(86) Internationale Anmeldenummer:
**PCT/EP2004/007734**

(87) Internationale Veröffentlichungsnummer:
**WO 2005/011545 (10.02.2005 Gazette 2005/06)**

(54) **VORRICHTUNG ZUM AUSBILDEN GEKRÜMMTER SCHNITTFLÄCHEN IN EINEM TRANSPARENTEN MATERIAL**

DEVICE FOR FORMING CURVED SECTIONS IN A TRANSPARENT MATERIAL

DISPOSITIF POUR FORMER DES SURFACES DE COUPE COURBES DANS UNE MATIERE TRANSPARENTE

(84) Benannte Vertragsstaaten:
**AT BE BG CH CY CZ DE DK EE ES FI FR GB GR HU IE IT LI LU MC NL PL PT RO SE SI SK TR**

(30) Priorität: **18.07.2003 DE 10332815**

(43) Veröffentlichungstag der Anmeldung:
**19.04.2006 Patentblatt 2006/16**

(73) Patentinhaber: **Carl Zeiss Meditec AG 07745 Jena (DE)**

(72) Erfinder:
• **MÜHLHOFF, Dirk**
  **07751 Kunitz (DE)**
• **GERLACH, Mario**
  **16548 Glienicke-Nordbahn (DE)**
• **STICKER, Markus**
  **07749 Jena (DE)**
• **LANG, Carsten**
  **07607 EISENBERG (DE)**
• **BISCHOFF, Mark**
  **07749 Jena (DE)**
• **BERGT, Michael**
  **99425 Weimar (DE)**

(74) Vertreter: **Geyer, Fehners & Partner Patentanwälte Perhamerstrasse 31 80687 München (DE)**

(56) Entgegenhaltungen:
WO-A-01/85075      US-A- 5 993 438
US-A- 6 110 166      US-A1- 2001 031 960

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 646 343 B1

**Beschreibung**

Die Erfindung bezieht sich auf eine Vorrichtung zum Ausbilden gekrümmter Schnittflächen in einem transparenten Material, insbesondere in der Augenhornhaut, mit einer gepulsten Laserstrahlungsquelle, die Laserstrahlung in das Material fokussiert und dort optische Durchbrüche bewirkt, wobei eine Ablenkeinrichtung, die die Laserstrahlung zweidimensional ablenkt und eine Steuereinrichtung vorgesehen sind, die die Ablenkeinrichtung ansteuert, um die Schnittfläche durch Aneinanderreihen der optischen Durchbrüche im Material zu bilden, wobei die Steuereinrichtung die Ablenkeinrichtung zweidimensional gemäß einer Ablenkfunktion so ansteuert, daß die Orte optischer Durchbrüche entlang einer Kurve, auf der die optischen Durchbrüche aneinandergereiht sind, gemäß einer auf die Ablenkung bezogenen Winkelfunktion beabstandet sind, die nichtlinear und so an die Krümmung der Schnittfläche angepaßt ist, daß die Orte entlang der Kurve benachbarter optischer Durchbrüche innerhalb einer bestimmten Toleranz in gleichmäßiger Distanz beabstandet sind.

Eine solche Vorrichtung ist aus der US 5993438 bekannt.

Gekrümmte Schnittflächen innerhalb eines transparentes Materials werden insbesondere bei laserchirurgischen Verfahren und dort insbesondere bei augenchirurgischen Eingriffen erzeugt. Dabei wird Behandlungs-Laserstrahlung innerhalb des Gewebes d.h. unterhalb der Gewebeoberfläche derart fokussiert, daß optische Durchbrüche im Gewebe entstehen.

Im Gewebe laufen dabei zeitlich hintereinander mehrere Prozesse ab, die durch die Laserstrahlung initiiert werden. Überschreitet die Leitungsdichte der Strahlung einen Schwellwert, kommt es zu einem optischen Durchbruch, der im Material eine Plasmablase erzeugt. Diese Plasmablase wächst nach Entstehen des optischen Durchbruches durch sich ausdehnende Gase. Wird der optische Durchbruch nicht aufrechterhalten, so wird das in der Plasmablase erzeugte Gas vom umliegenden Material aufgenommen, und die Blase verschwindet wieder. Dieser Vorgang dauert allerdings sehr viel länger, als die Entstehung der Blase selbst. Wird ein Plasma an einer Materialgrenzfläche erzeugt, die durchaus auch innerhalb einer Materialstruktur liegen kann, so erfolgt ein Materialabtrag von der Grenzfläche. Man spricht dann von Photoablation. Bei einer Plasmablase, die vorher verbundene Materialschichten trennt, ist üblicherweise von Photodisruption die Rede. Der Einfachheit halber werden all solche Prozesse hier unter dem Begriff optischer Durchbruch zusammengefaßt, d.h. dieser Begriff schließt nicht nur den eigentlichen optischen Durchbruch sondern auch die daraus resultierenden Wirkungen im Material ein.

Für eine hohe Genauigkeit eines laserchirurgischen Verfahrens ist es unumgänglich, eine hohe Lokalisierung der Wirkung der Laserstrahlen zu gewährleisten und Kolateralschäden in benachbartem Gewebe möglichst zu vermeiden. Es ist deshalb im Stand der Technik üblich, die Laserstrahlung gepulst anzuwenden, so daß der zur Auslösung eines optischen Durchbruchs nötige Schwellwert für die Leistungsdichte nur in den einzelnen Pulsen überschritten wird. Die US 5.984.916 zeigt diesbezüglich deutlich, daß der räumliche Bereich des optischen Durchbruchs (in diesem Fall der erzeugten Wechselwirkung) stark von der Pulsdauer abhängt. Eine hohe Fokussierung des Laserstrahls in Kombination mit sehr kurzen Pulsen erlaubt es damit, den optischen Durchbruch sehr punktgenau in einem Material einzusetzen.

Der Einsatz von gepulster Laserstrahlung hat sich in der letzten Zeit besonders zur laserchirurgischen Fehlsichtigkeitskorrektur in der Ophthalmologie durchgesetzt. Fehlsichtigkeiten des Auges rühren oftmals daher, daß die Brechungseigenschaften von Hornhaut und Linse keine ordnungsgemäße Fokussierung auf der Netzhaut bewirken.

Die erwähnte US 5.984.916 sowie die US 6.110.166 beschreiben gattungsgemäße Verfahren zur Schnitterzeugung mittels geeigneter Erzeugung optischer Durchbrüche, so daß im Endeffekt die Brechungseigenschaften der Hornhaut gezielt beeinflußt werden. Eine Vielzahl von optischen Durchbrüchen wird so aneinander gesetzt, daß innerhalb der Hornhaut des Auges ein linsenförmiges Teilvolumen isoliert wird. Das vom übrigen Hornhautgewebe getrennte linsenförmige Teilvolumen wird dann über einen seitlich öffnenden Schnitt aus der Hornhaut herausgenommen. Die Gestalt des Teilvolumens ist so gewählt, daß nach Entnahme die Brechungseigenschaften der Hornhaut so geändert sind, daß die erwünschte Fehlsichtigkeitskorrektur bewirkt ist. Die dabei geforderten Schnittflächen sind gekrümmt, was eine dreidimensionale Verstellung des Fokus nötig macht. Es wird deshalb üblicherweise eine zweidimensionale Ablenkung der Laserstrahlung mit gleichzeitiger Fokusverstellung kombiniert.

Zur Isolierung des Teilvolumens ist es natürlich unumgänglich, die optischen Durchbrüche an vorbestimmten Stellen zu erzeugen. Dabei hängt die Qualität der erzeugten Schnittfläche von der Gleichmäßigkeit der Anordnung der optischen Durchbrüche ab. Dies gilt insbesondere für die erwähnten augenchirurgischen, eine refraktive Korrektur vornehmenden Eingriffe, da dort die Qualität der Schnittfläche untrennbar mit der optischen Qualität des erzielten Ergebnisses verbunden ist.

Um die gekrümmten Schnittflächen mit hoher Qualität zu erzeugen, ist es deshalb unerläßlich, die optischen Durchbrüche mit hoher Dichte aneinanderzureihen. Dies ist jedoch einer schnellen Schnitterzeugung aus zwei Gründen hinderlich: Zum einen steigt die Dauer für die Erzeugung der Schnittfläche mit der erforderlichen Anzahl an optischen Durchbrüchen. Zum anderen muß bei einer engen Aneinanderreihung von optischen Durchbrüchen nach einem jeden Durchbruch abgewartet werden, bis die in der Plasmablase erzeugte Gasmenge wieder vom umliegenden Gewebe aufgenommen wurde, bevor unmittelbar benachbart der nächste optische Durchbruch erzeugt werden kann. Ansonsten könnte der optische Durchbruch nicht mit ausreichender Sicherheit erzeugt werden, da womöglich die Laserstrahlung in eine noch bestehende Plasmablase fokussiert würde und dort keinen optischen Durchbruch bewirkte.

Der Erfindung liegt deshalb die Aufgabe zugrunde, eine Vorrichtung zum Ausbilden gekrümmter Schnittflächen der eingangs genannten Art so fortzubilden, daß eine gute Qualität der optischen Schnittfläche bei gleichzeitig möglichst schneller Ausbildung der Schnittfläche möglich ist.

Die Aufgabe wird gelöst mit einer Vorrichtung gemäß Anspruch 1.

Die Erfindung geht von einer Projektionsgeometrie aus, bei der äquidistante Winkelabstände der Ablenkung in einer Ebene senkrecht zur Haupteinfallsachse äquidistant liegende Punkte zur Folge haben. Erfindungsgemäß wird durch nichtäquidistante Winkelabstände dafür gesorgt, daß die Punktabstände in der Ebene senkrecht zur Haupteinfallsachse so variieren, daß durch die gekrümmte Schnittfläche wieder äquidistante Orte der optischen Durchbrüche vorliegen.

Die Erfindung erreicht, daß in der gekrümmten Schnittfläche entlang einer Kurve benachbart gelegene optische Durchbrüche innerhalb genau bestimmter Grenzen äquidistant liegen. Der Abstand derart benachbarter optischer Durchbrüche kann nun so eingestellt werden, daß zum Erzeugen einer gewünschten gekrümmten Schnittfläche eine möglichst geringe Anzahl an optischen Durchbrüchen ausreicht. Weiter kann der Abstand zuverlässig so eingestellt werden, daß die optischen Durchbrüche mit möglichst hoher Frequenz aneinandergefügt werden können, ohne das die Gefahr besteht, einen optischen Durchbruch vergeblich in der noch nicht kollabierten Plasmablase des benachbarten optischen Durchbruches generieren zu wollen.

Bezüglich der Aneinanderreihung der Durchbrüche läßt die Erfindung großen Freiraum, wesentlich für den erfindungsgemäßen Ansatz ist es lediglich, daß die entlang einer Kurve benachbart aufgereihten Durchbrüche den oben erwähnten geometrischen Bedingungen genügen, wobei die Kurve mit der zeitlichen Reihenfolge, mit der die Durchbrüche erzeugt wurden, verknüpft sein kann und ihr insbesondere entsprechen kann.

Gleichzeitig erreicht das erfindungsgemäße Konzept, daß mit einer üblicherweise sehr einfach vorzunehmenden zweiachsigen Ablenkung für die die optischen Durchbrüche erzeugenden Laserstrahlung bearbeitet werden kann. Eine aufwendige zweidimensionale Verschiebung, bei der keine Ablenkung um feststehende Achsen erfolgt, wie es beispielsweise durch zweidimensionale Verschiebung eines Endes eines Lichtleiters erreicht werden könnte, ist mit dem erfindungsgemäßen Ansatz nicht erforderlich.

Als zweidimensionale Ablenkung im Sinne der Erfindung kommt jede Ablenkung in Frage, die die Laserstrahlung in zwei Richtungen ablenkt. Üblicherweise nimmt man mittels Kippspiegeln eine Ablenkung um zwei orthogonale Achsen vor. Die Achsen liegen dabei naturgemäß etwas beabstandet und senkrecht zur Haupteinfallsachse der Strahlung auf das Objekt (z.B. Auge). Durch Projektion der Achse der zuerst erfolgten Ablenkung entlang der Haupteinfallsachse auf die Achse der nachfolgenden Ablenkung erhält man eine Basis der zweidimensionalen Ablenkung. Generell müssen sich die Achsen also nicht schneiden; sie müssen in der erwähnten Projektion auch nicht orthogonal sein; z.B. kommt auch eine Ablenkung gemäß Kreiskoordinaten in Frage, wie sie unter Verwendung eines Taumelspiegels oder eines Drehprismenscanners realisiert werden kann.

In jedem Fall können immer zwei eine Ebene senkrecht zur Haupteinfallsachse aufspannende Basen angegeben werden, auf die die Winkelfunktion bezogen wird. Die Winkelfunktion ist natürlich an die Krümmung der Schnittfunktion angepaßt. Sie ist auf die zweidimensionale Ablenkung bezogen, gibt den Ort eines optischen Durchbruchs bezüglich der Ablenkung (Ablenkpunkt, Basen der Ablenkung) an und ist deshalb im allgemeinen auch zweidimensional. Je nach Art der Ablenkung kann diese Winkelfunktion in zwei eindimensionale Winkelfunktionen separierbar sein. Bei einer zweiachsigen Ablenkung läßt sich beispielsweise für jede Achse eine eigene Winkelfunktion angeben, die den Winkel zwischen der Ablenkachse und einer Geraden vom Kreuzungspunkt Strahlmittelachse-Ablenkachse zum Ort des optischen Durchbruches definiert. Der Kreuzungspunkt kann dabei als Ablenkpunkt für die Ablenkung um die betreffende Ablenkachse aufgefaßt werden. Ein Fachmann kann eine Vorgabe für die zu verwendende Winkelfunktion also z.B. dadurch erhalten, indem er äquidistant in der gekrümmten Schnittfläche liegende Orte mit dem Ablenkpunkt verbindet.

Die Erfindung erlaubt es, die Orte der entlang einer Kurve benachbarten optischen Durchbrüche mit genau bestimmtem Abstand zueinander durch die Ablenkung des Laserstrahls einzustellen. Da die Schnittfläche durch eine zweidimensionale Ablenkung erzeugt wird und üblicherweise die optischen Durchbrüche Plasmablasen mit meist kugelförmigen Volumen erzeugen, hängt der Abstand zwischen beliebig benachbarten Orten der optischen Durchbrüche in der Schnittfläche von der in einfacher Näherung als ebene Gitterstruktur auffaßbaren Anordnung der optischen Durchbrüche in der Schnittfläche ab. Der Abstand eines bestimmten optischen Durchbruches zu seinen Nachbarn schwankt je nach Gitterstruktur mehr oder weniger stark. Aufgrund dieser Schwankung ist es mitunter nicht immer zwingend erforderlich, entlang der Kurve sehr exakt ein und denselben Abstand zwischen den Orten einzustellen. Vielmehr genügt es, den Abstand innerhalb bestimmter Toleranzgrenzen konstant zu halten.

Um die Schnittfläche vollständig durch die kugelförmigen Plasmablasen zu füllen, wird man zweckmäßigerweise einen Abstand der Orte der optischen Durchbrüche in der Schnittfläche wählen, der über dem minimalen Abstand in der Gitterstruktur liegt. Vorteilhafterweise wird man die Toleranzgrenzen geringer wählen, als die geometrisch bedingte Variation der Abstände zwischen den Orten unmittelbar benachbarter optischer Durchbrüche. Als günstig hat sich eine Toleranz von 20% herausgestellt.

Die optischen Durchbrüche werden, wie eingangs bereits erwähnt, durch die Verwendung gepulster Laserstrahlung erzeugt. Durch die Ablenkung entstehen dabei um gewisse Schrittweiten beabstandet die optischen Durchbrüche. Auf die Schrittweite, d.h. Winkelabstände der optischen Durchbrüche, wirken sich gleichermaßen die Ablenkfunktion, gemäß der die Ablenkung erfolgt, und der zeitliche Abstand zwischen zwei aufeinanderfolgenden Pulsen, d.h. die Pulsfrequenz, aus. Beide Größen sind somit zur Einstellung der erfindungsgemäßen Winkelfunktion gleichermaßen tauglich, wobei ein Konstanthalten der einen unter Variation der anderen ebenso möglich ist, wie eine gleichzeitige Variation von Pulswiederholfrequenz- und Ablenkfunktion.

Als technisch besonders einfach zu realisieren erweist sich eine konstante Pulswiederholfrequenz bei der Bereitstellung der Laserstrahlung. Es ist deshalb als Weiterbildung zu bevorzugen, die Laserstrahlung gleichmäßig gepulst bereitzustellen und die Ablenkung gemäß einer mindestens in einer Richtung nichtlinearen Ablenkfunktion vorzunehmen, d.h. so zu gestalten, daß die Verstellgeschwindigkeit der zweidimensionalen Ablenkung vom gerade vorliegenden Ablenkwinkel abhängt, um die erfindungsgemäße Winkelfunktion zu realisieren. Dies gilt insbesondere für den mit besonders einfachen Mitteln zu realisierenden Fall einer Ablenkung um zwei senkrecht zueinanderliegender Achsen.

Mit einer solchen Ablenkung ist es insbesondere möglich, die Laserstrahlung in einem mäanderförmigen Muster über den Bereich, in dem die Schnittfläche erzeugt werden soll, zu führen. Der Laserstrahl wird dabei idealerweise in parallel liegenden Zeilen hin und her abgelenkt, und senkrecht dazu erfolgt am Ende einer jeweiligen Zeile eine Veränderung der Ablenkung um die andere Achse, um einen Zeilenvorschub zu erreichen. Je nach Ausbildung der Schnittfläche sind die Zeilen natürlich nicht gleich lang. Sie liegen weiter, um der Krümmung der Schnittfläche Rechnung zu tragen und die erfindungsgemäße nichtlineare Winkelfunktion zu erreichen, in der Regel auch nicht äquidistant zueinander; ihr Abstand variiert je nach Lage der Zeile auf der Schnittfläche. Bei bestimmten Schnittflächenformen kann es auch auftreten, daß die Ablenkung zur Verschiebung der Zeilen während des Fortschreitens auf einer Zeile verstellt werden muß, um die an die gekrümmte Schnittfläche angepaßte Winkelfunktion zu erhalten.

Um die gewünschte Krümmung der Schnittfläche zu erreichen, muß natürlich für eine entsprechende Verstellung der Fokussierung während der zweidimensionalen Ablenkung gesorgt werden. Das dazu erforderliche Vorgehen sowie die dazu erforderlichen Mittel sind dem Fachmann aus dem Stand der Technik jedoch bekannt, so daß hier nicht weiter darauf eingegangen werden muß. Es genügt hier der Hinweis, daß die Verstellung des Fokuspunktes gemäß der zu erreichenden gekrümmten Schnittfläche mit der zweidimensionalen Ablenkung geeignet synchronisiert wird, so daß jeder Ablenkung eine Fokuslage zugeordnet ist.

Bei augenchirurgischen Eingriffen werden, da die Augenhornhaut sphärisch gekrümmt ist, meist annähernd sphärisch oder sphärisch mit einem zylindrischen Anteil gekrümmte Schnittflächen gefordert. Insbesondere für solche Schnittflächen ist bei gleichmäßig gepulster Laserstrahlung eine Ablenkung vorteilhaft, bei der die Ablenkgeschwindigkeit am Rand des Bereiches der Schnittfläche geringer ist, als im Zentrum, um der am Rand des Bereiches zunehmenden Neigung der gekrümmten Schnittfläche gegenüber der Ebene der zweidimensionalen Ablenkung Rechnung zu tragen.

Bei sphärisch gekrümmten Schnittflächen läßt sich die in einer Ebene parallel zur zweidimensionalen Ablenkung auftretende Schrittweite zwischen benachbarten Durchbrüchen besonders einfach exakt einstellen. Es ist für mit Radius R gekrümmte Schnittflächen diesbezüglich eine Weiterbildung bevorzugt, bei der die Laserstrahlung auf eine Haupteinfallsachse in das Material einfällt und in einer Ebene senkrecht zu dieser Haupteinfallsachse zweiachsig entlang einer x- und einer y-Achse abgelenkt wird, wobei in der Ebene in x-Richtung eine Schrittweite dx zwischen Orten auf der Kurve benachbarter optischer Durchbrüche eingestellt wird, für die gilt:

$$dx = D \bullet \frac{R1}{\sqrt{R1^2 - x^2}},$$

wobei D die Distanz der optischen Durchbrüche (8) bezeichnet und

$$R1 = R \bullet \cos(\arctan\frac{y}{R})$$

ist.

Analog kann die Pulsfrequenz der Laserstrahlung am Rand des Bereiches der Schnittfläche höher gewählt werden, als im Zentrum.

Das geschilderte mäanderförmige Muster ist ein Spezialfall einer zweidimensionalen Ablenkung mit zweier den Koordinaten der zweidimensionalen Ablenkung zugeordneten Ablenkfunktionen, wobei eine der zwei Ablenkfunktionen mit der Koordinate, mit der die andere der zwei Ablenkfunktionen zugeordnet ist parametrisiert ist. Eine solche Weiterbildung der Erfindung ermöglicht die Verwendung weitgehend separierter Ablenkfunktionen und vermeidet den Aufwand von an und für sich natürlich möglichen zweidimensionalen Ablenkfunktionen, die datentechnisch sehr viel schwerer zugänglich sind.

Die erfindungsgemäße Vorrichtung dient zur Realisierung des Verfahrens und weist eine diesbezüglich ausgebildete Steuereinrichtung auf, die beispielsweise mit einem geeignet programmierter Computer verwirklicht werden kann und die die Ablenkeinrichtung sowie weitere Komponenten der Vorrichtung (z.B. Fokusverstellung) steuert. Eine besonders zweckmäßige Realisierung für die Projektion der Laserstrahlung in das Material ist eine korrigierte F-Theta-Optik, die bei der Ablenkung aus einem Ablenkpunkt heraus bei einer linearen Ansteuerung der Ablenkung zu einem gleichmäßigen Verlauf der Ablenkung parallel zur Ebene der zweidimensionalen Ablenkung führt.

Die Erfindung wird nachfolgend unter Bezugnahme auf die Zeichnung beispielhalber noch näher erläutert. In der Zeichnung zeigt:

Fig. 1 eine perspektivische Darstellung eines Patienten während einer laserchirurgischen Behandlung mit einem laserchirurgischen Instrument,

Fig. 2 die Fokussierung eines Strahlenbündels auf das Auge des Patienten beim Instrument der Figur 1,

Fig. 3 eine schematische Darstellung zur Erläuterung einer während der laserchirurgischen Behandlung mit dem Instrument der Figur 1 erzeugten Schnittfläche,

Fig. 4 eine Ablenkvorrichtung des laserchirurgischen Instruments der Figur 1,

Fig. 5 die Anordnung von optischen Durchbrüchen zur Erzeugung einer Schnittfläche mit dem laserchirurgischen Instrument der Figur 1,

Fig. 6 eine Kurve zur Veranschaulichung der Zeilenablenkung bei der Ablenkfunktion der Figur 4,

Fig. 7 weitere Kurven zur Ansteuerung der Ablenkvorrichtung der Figur 4 und

Fig. 8 zwei Zeitreihen zur Ansteuerung der zweiachsigen Ablenkung gemäß Figur 4.

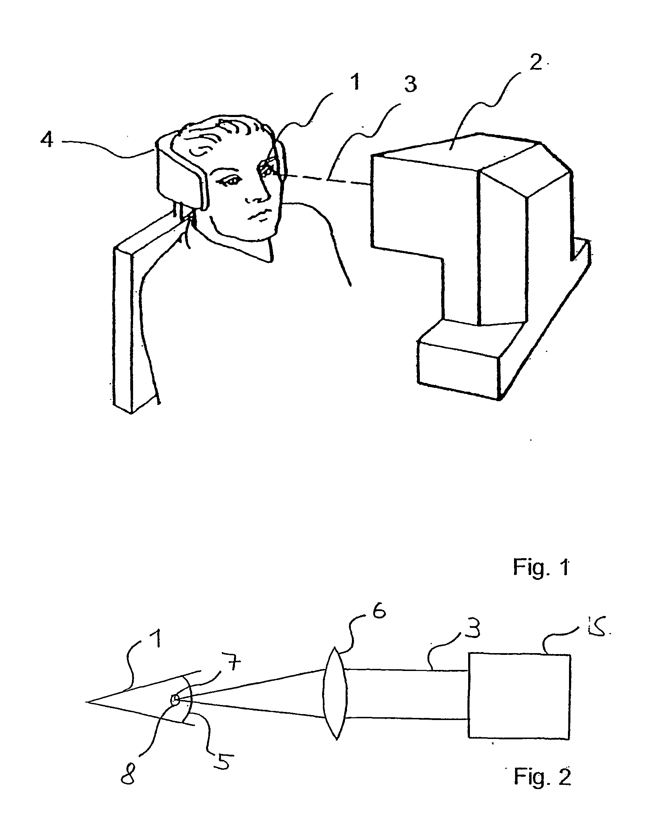

In Figur 1 ist ein laserchirurgisches Instrument zur Behandlung eines Auges 1 eines Patienten gezeigt, wobei das laserchirurgische Instrument 2 zur Ausführung einer refraktiven Korrektur dient. Das Instrument 2 gibt dazu einen Behandlungs-Laserstrahl 3 auf das Auge des Patienten 1 ab, dessen Kopf in einen Kopfhalter 4 fixiert ist. Das laserchirurgische Instrument 2 ist in der Lage, einen gepulsten Laserstrahl 2 zu erzeugen, so daß das in US 6.110.166 beschriebene Verfahren ausgeführt werden kann.

Das laserchirurgische Instrument 2 weist dazu, wie in Figur 2 schematisch dargestellt ist, eine Strahlquelle S auf, deren Strahlung in die Hornhaut 5 des Auges 1 fokussiert wird. Mittels des laserchirurgischen Instrumentes 2 wird eine Fehlsichtigkeit des Auges 1 des Patienten dadurch behoben, daß aus der Hornhaut 5 Material so entfernt wird, daß sich die Brechungseigenschaften der Hornhaut um ein gewünschtes Maß ändern. Das Material wird dabei dem Stroma der Hornhaut entnommen, das unterhalb von Epithel und Bowmanscher Membran oberhalb der Decemetschen Membran und des Endothels liegt.

Die Materialentfernung erfolgt, indem durch Fokussierung des hochenergetischen gepulsten Laserstrahls 3 mittels einer Fokussieroptik 6 in einem in der Hornhaut 5 liegenden Fokus 7 in der Hornhaut Gewebeschichten getrennt werden. Jeder Puls der gepulsten Laserstrahlung 3 erzeugt dabei einen optischen Durchbruch im Gewebe, welcher eine Plasmablase 8 initiiert. Dadurch umfaßt die Gewebeschichttrennung ein größeres Gebiet, als der Fokus 7 der Laserstrahlung 3. Durch geeignete Ablenkung des Laserstrahls 3 werden nun während der Behandlung viele Plasmablasen 8 aneinandergereiht. Die aneinanderliegenden Plasmablasen 8 bilden dann eine Schnittfläche 9, die ein Teilvolumen T des Stromas, nämlich das zu entfernende Material der Hornhaut 5 umschreiben.

Das laserchirurgische Instrument 2 wirkt durch die Laserstrahlung 3 wie ein chirurgisches Messer, das, ohne die Oberfläche der Hornhaut 5 zu verletzen, direkt Materialschichten im Inneren der Hornhaut 5 trennt. Führt man den Schnitt durch weitere Erzeugung von Plasmablasen 8 bis an die Oberfläche der Hornhaut 5, kann ein durch die Schnittfläche 9 isoliertes Material der Hornhaut 5 seitlich herausgezogen und somit entfernt werden.

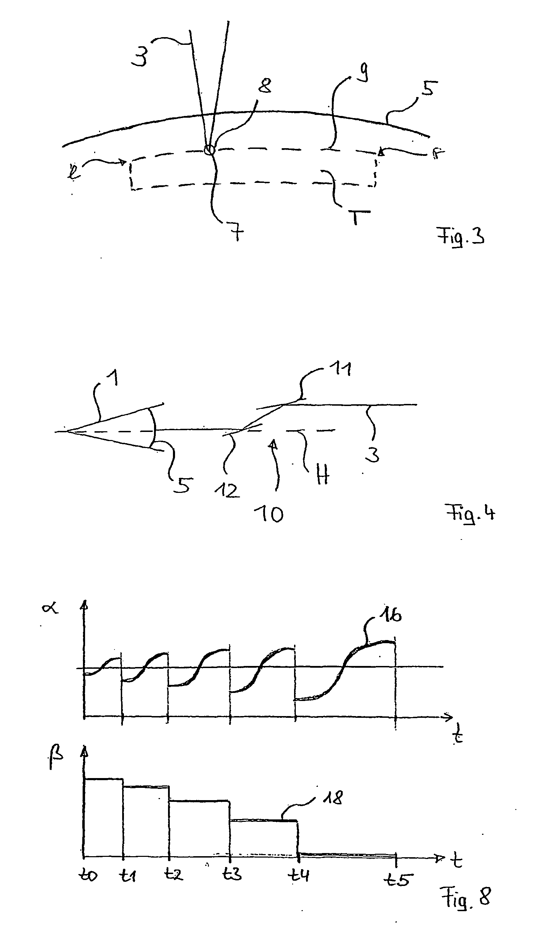

Die Erzeugung der Schnittfläche 9 mittels des laserchirurgischen Instrumentes 2 ist in Figur 3 schematisch dargestellt. Durch Aneinanderreihung der Plasmablasen 8 in Folge stetiger Ablenkung des gepulsten fokussierten Laserstrahls 3 und geeigneter Fokusverstellung wird die Schnittfläche 9 gebildet.

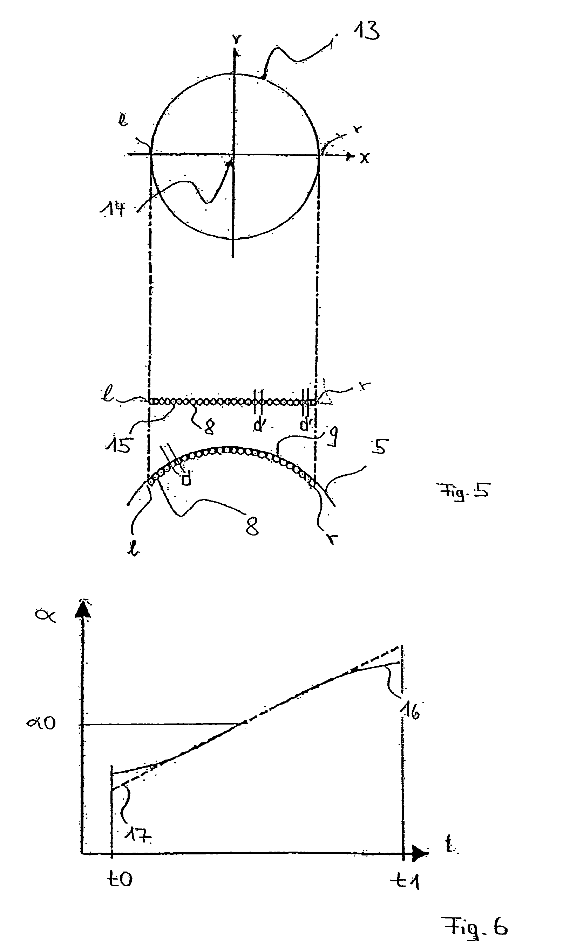

Die Ablenkung erfolgt dabei in einer Ausführungsform mittels der in Figur 4 schematisch dargestellten Ablenkeinheit 10, die den auf einer Haupteinfallsachse H auf das Auge 1 einfallenden Laserstrahls 3 um zwei senkrecht zueinander liegenden Achsen ablenkt. Die Ablenkeinheit 10 verwendet dafür einen Zeilenspiegel 11 sowie einen Bildspiegel 12, was zu zwei hintereinander liegenden räumlichen Ablenkachsen führt. Der Kreuzungspunkt der Hauptstrahlachse mit der Ablenkachse ist dann der jeweilige Ablenkpunkt. Da die beiden Ablenkpunkte für die zwei Ablenkachsen jedoch bezogen auf den Abstand zwischen Ablenkeinheit 10 und Hornhaut 5 sehr nahe beabstandet sind, können die Ablenkachsen näherungsweise als sich in einem einzigen Ablenkpunkt kreuzend angesehen werden. In der Näherung kann also davon ausgegangen werden, daß die beiden Ablenkachsen sich im Ablenkpunkt schneiden und so eine Ebene aufspannen, zu der die Haupteinfallsachse H senkrecht liegt. Sollte diese Näherung nicht möglich sein, sind die Ablenkachsen in eine Ebene senkrecht zur Haupteinfallsachse des Strahls auf die Hornhaut zu projizieren, um den Bezug für die noch zu erläuternde Winkelfunktion zu halten. Figur 5 zeigt in einer Draufsicht den Schnittbereich 13 eines in der Hornhaut 5 auszuführenden Schnittes, der sphärisch gekrümmt ist. Auf den Schnittbereich 13 ist die Projektion der beiden Ablenkachsen eingezeichnet, die sich im Zentrum 14 des Schnittbereiches schneiden und mit x bzw. mit y bezeichnet sind. Die Ablenkung durch den Zeilenspiegel 11 bewirkt dabei eine Verschiebung des Laserstrahls entlang der x-Achse (d.h. eine Ablenkung um die y-Achse) wohingegen die Betätigung des Bildspiegels 12 eine Verschiebung entlang der y-Achse (d.h. eine Ablenkung um die x-Achse) zur Folge hat.

Der Schnittbereich verläuft dabei von einem linken Rand I mit den Koordinaten x=-x$_{min}$, y=0, bis zu einem rechten Rand r mit den Koordinaten x=x$_{max}$, y=0. Die Ränder I, r sind zur Verdeutlichung in Figur 3 noch einmal eingetragen. Neben der Draufsicht des Schnittbereiches 13 zeigt Figur 5 unten eine Schnittdarstellung der Hornhaut 5 mit dem Schnittbereich 13, wobei der Schnitt entlang der x-Achse eingezeichnet ist. Wie zu sehen ist, wird die Schnittfläche 9 in der Hornhaut 5 durch Aneinanderreihung von Plasmablasen 8 erzeugt, die entlang der Schnittfläche 9 äquidistant mit Abstand d beabstandet sind. Der Abstand d ist abhängig vom Durchmesser der mit einem optischen Durchbruch erzeugten Plasmablase 8 und beträgt beispielsweise 3μm.

Zwischen der Schnittdarstellung unten in Figur 5 und der Draufsicht auf den Schnittbereich 13 Figur 5 oben ist die Aneinanderreihung der Plasmablasen 8 in Form einer Zeile 15 in einer Ebene senkrecht zur Haupteinfallsachse der Ablenkachsen dargestellt. Die Haupteinfallsachse ist dabei durch den auf das Zentrum 14 fallenden Strahl definiert. Wie zu sehen ist, schwankt der Abstand d' der einzelnen Plasmablasen 8 in dieser Ebene; der Abstand d' sinkt zu den Rändern I und r hin und ist im Zentrum, d.h. auf der Haupteinfallsachse bei y=0 maximal. Die Abnahme des Abstandes d' ist durch eine nichtlineare Ansteuerung des Zeilenspiegels 11 bewirkt und so gewählt, daß sich in der Schnittfläche 9 der Hornhaut 5 der gleichmäßige Abstand d zwischen den Plasmablasen 8 einstellt.

Bezogen auf den Ablenkpunkt sind die Orte der Disruptionsblasen folglich gemäß einer nichtlinearen Winkelfunktion angeordnet. Die Winkelfunktion in x-Richtung ist so gewählt, daß trotz Krümmung der Schnittfläche 9 die Plasmablasen 8 in der Schnittfläche 9 äquidistant liegen. Der infolge der Winkelfunktion variierende Abstand d' entlang der (geraden) Zeile 15 bewirkt also quasi eine Vorverzerrung der Abstände, die zusammen mit der Krümmung der Schnittfläche 9 den konstanten Abstand d der Orte der Plasmablasen 8, die entlang der Zeile geschrieben wurden, erreicht.

Die Orte in der linearen Zeile sind dabei x-abhängig zur Ausführung eines sphärisch gekrümmten Schnittes, mit Radius R wie folgt um d'=dx beabstandet:

$$dx = D \bullet \frac{R1}{\sqrt{R1^2 - x^2}},$$

wobei D die Distanz der optischen Durchbrüche (8) bezeichnet und

$$R1 = R \bullet \cos(\arctan\frac{y}{R})$$

ist.

Die für die Zeile 15 erforderliche Ansteuerung des Zeilenspiegels 11 ist in Figur 6 als Zeitreihe eingetragen. Die Zeitreihe der Figur 6 zeigt den Verlauf des Ablenkwinkels α als zeitenabhängige Zeilenfunktion 16 beim Schreiben der Zeile 15, d.h. beim Erzeugen der Plasmablasen 8 zur Koordinate y=0. Figur 6 zeigt dabei den Zeilen-Äblenkwinkel α, der aufgrund der gleichmäßig gepulst abgebende Strahlquelle S und der F-Theta-Korrektur der Optik identisch ist mit der Zeilenkoordinate x. Bei ungleichmäßiger gepulster Laserstrahlung gilt diese Zuordnung nicht mehr, weshalb zur Allgemeinheit der Darstellung in Figur 6 der letztlich ausschlaggebende Zeilen-Ablenkwinkel α aufgetragen ist. Um zu verdeutlichen, daß die Zeilenfunktion 16 nichtlinear ist, ist in Figur 6 zusätzlich der lineare Verlauf 17 aufgetragen, der aber bei der Ansteuerung des Zeilenspiegels 11 nicht verwendet wird.

Wie Figur 6 zeigt, wird der Zeilenspiegel 11 zwischen zwei Zeitpunkten t0 und t1 gemäß einer Zeilenfunktion 16 angesteuert, die symmetrisch um einen Mittelwert α0 liegt und von einer linearen Funktion 17 abweicht. Zum Zeitpunkt t0 steht der Zeilenspiegel 11 dabei so, daß der Laserstrahl 3 auf den linken Rand I fällt. Zum Zeitpunkt t1 befindet sich der abgelenkte Laserstrahl 3 am rechten Rand r. Der Wert α0 entspricht dem Zentrum des Schnittbereiches 13, d.h. der Koordinate x=0. Durch die zum Rand hin abflachende Zeilenfunktion 16 bewegt sich der Zeilenspiegel 11 am Rand des Schnittbereiches 13 mit geringerer Geschwindigkeit, wodurch die Plasmablasen 8 näher aneinander rücken, d.h. der Abstand d' sinkt zu den Rändern I und r hin. Aufgrund der Krümmung der Schnittfläche 9 ist damit insgesamt erreicht, daß der Abstand d der Plasmablasen 8 in der Hornhaut 5 konstant ist.

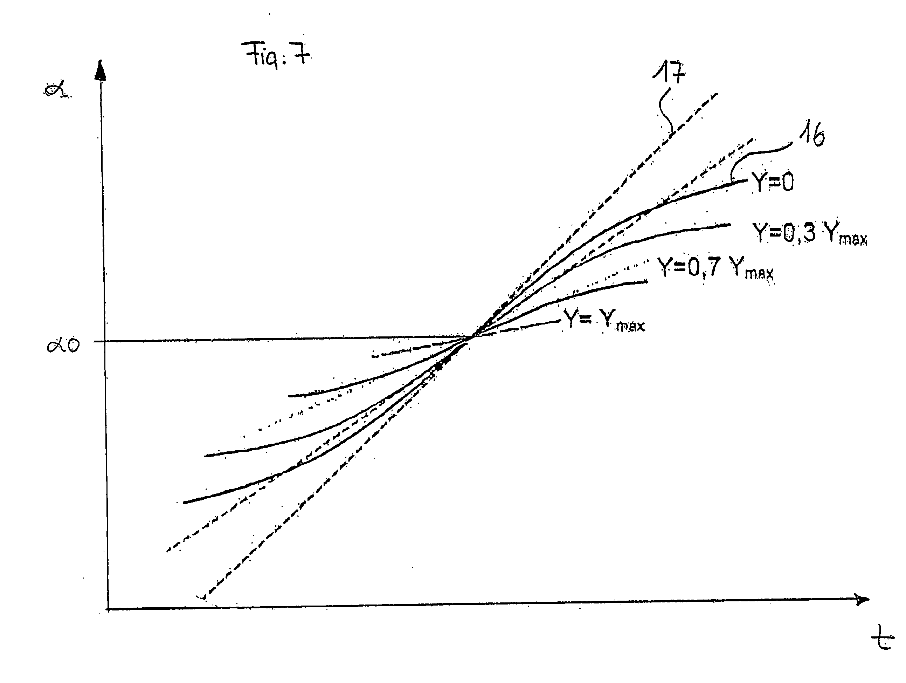

Die Zeile 15 der Figur 5 sowie die Zeilenfunktion 16 sind in Figur 6 für den vereinfachten Fall einer durch das Zentrum 14 laufenden Ablenkung dargestellt. Um die sphärische Schnittfläche, die in der Darstellung der Figur 5 in der Draufsicht einen kreisförmigen Schnittbereich 13 zur Folge hat, durch Zeilenverschiebung zu erreichen, muß natürlich die maximale Auslenkung des Zeilenspiegels 11 an die aktuelle Stellung des Bildspiegels 12 angepaßt werden. Dies hat zur Folge, daß die Zeilenfunktion 16 hier mit dem y-Wert, d.h. mit der Stellung des Bildspiegels 12, parametrisiert ist. Figur 7 zeigt ein Beispiel für eine entsprechende Kurvenschar an Zeilenfunktionen 16. Die Steigung der Zeilenfunktion 16 nimmt mit zunehmendem y-Wert ab, gleichzeitig nimmt der Zeilenöffnungswinkel, d.h. die Differenz zwischen maximalem und minimalem Zeilenablenkwinkel α ab. Die Gestaltung hängt im Einzelfall natürlich von der Schnittfläche 9 und der Korrektur der Optik ab.

Der Zeilenspiegel 11 wird also mit einer Ansteuerfunktion angesteuert, deren Verlauf von der Stellung des Bildspiegels 12 abhängt. Dies ist in Figur 8 noch einmal verdeutlicht, die in zwei Zeitreihen den Verlauf des Zeilen-Ablenkwinkels α (im Ausführungsbeispiel identisch mit oder proportional zur der x-Koordinate) und eines Bild-Ablenkwinkels β wiedergibt, der bei der beschriebenen Ausführungsform mit konstant gepulster Strahlquelle S identisch mit der oder proportional zur y-Koordinate ist. In den in Figur 8 dargestellten Zeitreihen wird der Schnittbereich 13 von oben nach unten abgerastert. Zum Zeitpunkt t0 ist der Bildspiegel 13 konstant auf eine Ablenkung am oberen Rand des Schnittbereichs 13 gestellt, wie die Bildfunktion 18 zeigt. Der Zeilenspiegel 11 wird dagegen über einen kleinen Bereich nichtlinear verstellt. Der zeitliche Verlauf der Ansteuerfunktion und damit des Ablenkwinkels hängt von der Krümmung der Schnittfläche ab.

Ist die Verstellung zum Zeitpunkt t1 abgeschlossen, rückt der Bildspiegel 12 gemäß der Bildfunktion 18 in die nächste Zeile, und der Zeilenspiegel führt wiederum eine nichtlineare Zeilenbewegung aus, wobei er zuerst wieder an den linken Rand des Schnittbereichs 13. zurückgeführt wurde. Die nun bewirkte Ablenkung mittels des Zeilenspiegels 18 erfolgt ebenfalls nichtlinear, jedoch über einen gegenüber der vorherigen Ablenkung vergrößerten Zeilenöffnungswinkel. Ist die Ablenkung entlang der zweiten Zeile abgeschlossen, wird der Bildspiegel zum Zeitpunkt t2 in die dritte Zeile gestellt, wobei die dabei bewirkte Winkeländerung größer ist, als bei den vorherigen Zeilen, da benachbarte Zeilen nicht winkeläquidistant liegen. Dieses Vorgehen ist in Fig. 8 bis zu der Zeile 15 der Fig. 5 dargestellt, wobei zur starken Vereinfachung lediglich 5 Zeilen in Fig. 8 enthalten sind.

Anstelle des Rücksprungs des Zeilenspiegels 11 können die Zeilen auch mäanderförmig aneinandergereiht werden, die Zeilenfunktion 16 ist dann zwischen den Abschnitten t1 und t2 sowie t3 und t4 invertiert.

**Patentansprüche**

1. Vorrichtung zum Ausbilden gekrümmter Schnittflächen (9) in einem transparenten Material, insbesondere in der Augenhornhaut (5), mit einer gepulsten Laserstrahlungsquelle (S), die Laserstrahlung (3) in das Material (5) fokussiert und dort optische Durchbrüche (8) bewirkt, wobei eine Ablenkeinrichtung (10), die die Laserstrahlung (3) zweidimensional ablenkt, und eine Steuereinrichtung (2) vorgesehen sind, die die Ablenkeinrichtung (10) ansteuert, um die Schnittfläche (9) durch Aneinanderreihen der optischen Durchbrüche (8) im Material (5) zu bilden, wobei die Steuereinrichtung (2) die Ablenkeinrichtung (10) zweidimensional gemäß einer Ablenkfunktion (16, 17) so ansteuert, daß die Orte optischer Durchbrüche (8) entlang einer Kurve, auf der die optischen Durchbrüche (8) aneinandergereiht sind, gemäß einer auf die Ablenkung bezogenen Winkelfunktion beabstandet sind, die nichtlinear und so an die Krümmung der Schnittfläche (9) angepaßt ist, daß die Orte entlang der Kurve benachbarter optischer Durchbrüche (8) innerhalb einer bestimmten Toleranz in gleichmäßiger Distanz (D) beabstandet sind, **dadurch gekennzeichnet**, daß am Rande eines Bereiches (13), in dem die Schnittfläche (9) ausgebildet wird, in einer Dimension (x) die Ablenkung mit geringerer Geschwindigkeit erfolgt als im Zentrum (14) des Bereiches (13).

2. Vorrichtung nach Anspruch 1, **dadurch gekennzeichnet, daß** die Toleranz 20% beträgt.

3. Vorrichtung nach einem der obigen Vorrichtungsansprüche **dadurch gekennzeichnet, daß** die Laserstrahlungsquelle (S) gleichmäßig gepulste Laserstrahlung (3) abgibt und die Steuereinrichtung (2) die Ablenkeinrichtung (10) in beiden Dimensionen (x, y) gemäß einer nichtlinearen Ablenkfunktion (16, 17) ansteuert.

4. Vorrichtung nach einem der obigen Vorrichtungsansprüche, **dadurch gekennzeichnet, daß** die Ablenkung um zwei senkrecht zueinanderliegende Achsen (x, y) erfolgt, wobei die Steuereinrichtung (2) die Laserstrahlung (3) entlang eines mäanderförmigen Musters führt.

5. Vorrichtung nach einem der obigen Vorrichtungsansprüche, **dadurch gekennzeichnet, daß** die Schnittfläche (9) sphärisch mit Radius R gekrümmt ist, die Laserstrahlung (3) auf eine Haupteinfallsachse (H) in das Material (5) einfällt und die Ablenkungseinrichtung (10) die Laserstrahlung (3) in einer Ebene senkrecht zu dieser Haupteinfallsachse (H) zweiachsig entlang einer x- und einer y-Achse ablenkt, wobei die Steuereinrichtung (2) in der Ebene in x-Richtung eine Schrittweite dx zwischen Orten auf der Kurve benachbarter optischer Durchbrüche (8) einstellt, für die gilt:

$$dx = D \bullet \frac{R1}{\sqrt{R1^2 - x^2}},$$

wobei D die Distanz der optischen Durchbrüche (8) bezeichnet und

$$R1 = R \bullet \cos\!\left(\arctan\frac{y}{R}\right)$$

ist.

6. Vorrichtung nach einem der obigen Vorrichtungsansprüche, **dadurch gekennzeichnet, daß** die Steuereinrichtung (2) am Rande eines Bereiches (13), in dem die Schnittfläche (9) ausgebildet wird, die Pulsfrequenz der Laserstrahlung (3) verschieden von der im Zentrum (14) der Schnittfläche, insbesondere höher, steuert.

7. Vorrichtung nach einem der obigen Vorrichtungsansprüche, **dadurch gekennzeichnet, daß** die zweidimensionale Ablenkung gemäß zweier den Koordinaten der zweidimensionalen Ablenkung zugeordneten Ablenkfunktionen (16, 17) erfolgt, wobei eine der zwei Ablenkfunktionen (16) mit der Koordinate, der die andere der zwei Ablenkfunktionen (17) zugeordnet ist, parameterisiert ist.

**Claims**

1. Apparatus for producing curved cuts (9) in a transparent material, in particular in the cornea (5), said apparatus comprising a pulsed laser radiation source (S) which focuses laser radiation (3) into the material (5) and causes optical breakthroughs (8) there, wherein a deflecting unit (10) deflecting the laser radiation (3) two-dimensionally and a control unit (2) controlling said deflecting unit (10) are provided so as to form the cut (9) by sequential arrangement of the optical breakthroughs (8) in the material (5), wherein the control unit (2) controls the deflecting unit (10) two-dimensionally according to a deflection function (16, 17) such that the locations of optical breakthroughs (8) along a curve on which the optical breakthroughs (8) are sequentially arranged are spaced apart according to a deflection-related angular function, which is non-linear and adapted to the curvature of the cut (9) such that the locations of adjacent optical breakthroughs (8) along the curve are spaced apart by the same distance (D) within a certain tolerance, **characterised in that** at the periphery of a region (13) in which the cut (9) is produced, deflection in one dimension (x) is effected at a lower speed than at the centre (14) of the region (13).

2. Apparatus according to claim 1, **characterised in that** the tolerance is 20%.

3. Apparatus according to any one of the above apparatus claims, **characterised in that** the laser radiation source (S) emits uniformly pulsed laser radiation (3), and the control unit (2) controls the deflecting unit (10) in both dimensions (x, y) according to a non-linear deflection function (16, 17).

4. Apparatus according to any one of the above apparatus claims, **characterised in that** the deflection is effected about two mutually perpendicular axes (x, y), wherein the control unit (2) guides the laser radiation (3) in a meandering pattern.

5. Apparatus according to any one of the above apparatus claims, **characterised in that** the cut (9) is curved spherically with a radius R, the laser radiation (3) is incident in the material (5) along a main axis of incidence (H), and the deflecting unit (10) deflects the laser radiation (3) biaxially along an x-axis and a y-axis in a plane perpendicular to said main axis of incidence (H), wherein the control unit (2) sets a step width dx between locations on the curve of adjacent optical breakthroughs (8) in the plane in the x-direction according to:

$$dx = D \bullet \frac{R1}{\sqrt{R1^2 - x^2}},$$

wherein D designates the distance of the optical breakthroughs (8) and

$$R1 = R \bullet \cos\!\left(\arctan\frac{y}{R}\right)$$

6. Apparatus according to any one of the above apparatus claims, **characterised in that**, at the periphery of a region (13) in which the cut (9) is produced, the control unit (2) controls the pulse rate of the laser radiation (3) differently from, in particular higher than, the rate at the centre (14) of the cut.

7. Apparatus according to any one of the above apparatus claims, **characterised in that** the two-dimensional deflection is effected according to two deflection functions (16, 17) assigned to the coordinates of the two-dimensional deflection, wherein one of said two deflection functions (16) is parametrised with the coordinate to which the other of said two deflection functions (17) is assigned.

**Revendications**

1. Dispositif permettant de réaliser des surfaces de coupe (9) courbes dans une matière transparente, en particulier dans la cornée (5), comportant une source de rayonnement laser (S) pulsé, qui focalise un rayonnement laser (3) dans la matière (5) et y produit des percées optiques (8), et il est prévu un dispositif de déviation (10), qui dévie de manière bidimensionnelle le rayonnement laser (3), et un dispositif de commande (2), qui active le dispositif de déviation (10) pour former la surface de coupe (9) par la juxtaposition des percées optiques (8) dans la matière (5), le dispositif de commande (2) activant le dispositif de déviation (10) de manière bidimensionnelle selon une fonction de déviation (16, 17), de telle sorte que les points des percées optiques (8) sont écartés les uns des autres le long d'une courbe, sur laquelle sont juxtaposées les percées optiques (8), selon une fonction angulaire rapportée à la déviation, qui n'est pas linéaire et est ajustée à la courbure de la surface de coupe (9), de telle sorte que les points le long de la courbe des percées optiques (8) adjacentes sont écartés selon une distance (D) régulière à l'intérieur d'une tolérance déterminée, **caractérisé en ce que** sur le bord d'une zone (13), dans laquelle sera réalisée la surface de coupe (9), est effectuée une déviation dans une dimension (x) avec une vitesse plus faible que dans le centre (14) de ladite zone (13).

2. Dispositif selon la revendication 1, **caractérisé en ce que** la tolérance est de l'ordre de 20 %.

3. Dispositif selon l'une quelconque des revendications précédentes, **caractérisé en ce que** la source de rayonnement laser (S) émet un rayonnement laser (3) pulsé régulièrement et le dispositif de commande (2) active le dispositif de déviation (10) dans les deux dimensions (x, y) selon une fonction de déviation (16, 17) non linéaire.

4. Dispositif selon l'une quelconque des revendications précédentes, **caractérisé en ce que** la déviation est effectuée selon deux axes (x, y) perpendiculaires entre eux, le dispositif de commande (2) guidant le rayonnement laser (3) le long d'un modèle en forme de méandres.

5. Dispositif selon l'une quelconque des revendications précédentes, **caractérisé en ce que** la surface de coupe (9) a une courbure sphérique avec un rayon R, le rayonnement laser (3) est projeté sur un axe d'incidence principal (H) dans la matière (5), et le dispositif de déviation (10) dévie le rayonnement laser (3) en deux axes le long d'un axe x et d'un axe y dans un plan perpendiculaire à cet axe d'incidence principal (H), le dispositif de commande (2) réglant dans le plan dans la direction x un incrément dx entre les points sur la courbe des percées optiques (8) adjacentes, pour lequel on applique :

$$dx = D * \frac{R1}{\sqrt{R1^2 - x^2}},$$

dans laquelle D désigne la distance entre les percées optiques (8) et

$$R1 = R * \cos(\arctan \frac{y}{R})$$

6. Dispositif selon l'une quelconque des revendications précédentes, **caractérisé en ce que** sur le bord d'une zone (13), dans laquelle sera réalisée la surface de coupe (9), le dispositif de commande (2) commande la fréquence d'impulsion du rayonnement laser (3) différemment, en particulier plus élevée, de celle dans le centre (14) de la surface de coupe.

7. Dispositif selon l'une quelconque des revendications précédentes, **caractérisé en ce que** la déviation bidimensionnelle est effectuée selon deux fonctions de déviation (16, 17) associées aux coordonnées de la déviation bidimensionnelle, l'une des deux fonctions de déviation (16) étant paramétrée avec la coordonnée à laquelle est associée l'autre des deux fonctions de déviation (17).

Fig. 1

Fig. 2

Fig. 3

Fig. 4

Fig. 8

Fig. 5

Fig. 6

Fig. 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5993438 A
- US 5984916 A
- US 6110166 A